# EUROPEAN PATENT APPLICATION

(11) **EP 4 458 334 A1**
(43) Date of publication of application: **06.11.2024**
(21) Application number: 23171255.5
(22) Date of filing: 03.05.2023
(51) Int. Cl.: A61F 13/06

(54) **A PRESSURE OFFLOADING PAD FOR USE IN A GARMENT**

(71) Applicant: Mölnlycke Health Care AB, 402 52 Göteborg (SE)
(72) Inventor: JAKOBSSON, Conny, 443 38 LERUM (SE)
(74) Representative: Kransell & Wennborg KB

(57) **Abstract**

The present disclosure relates to a pressure offloading pad (100) for incorporation into a garment (200); the said pressure offloading pad (100) comprising a skin-facing layer (101) and a bladder (102), wherein the bladder comprises a pressure offloading material (103) selected from air or a fluidized medium. The present disclosure also relates to a garment (200) comprising the pressure offloading pad (100).

## Description

### TECHNICAL FIELD

The present invention relates to a pressure offloading pad for incorporation into a garment, wherein the pressure offloading pad comprises a skin-facing layer and a bladder comprising a pressure offloading material. The present invention also relates to a garment comprising the pressure offloading pad.

### BACKGROUND

Pressure ulcers often arise among persons being bedridden for various reasons, such as for instance due to long term hospitalization or other causes of immobility. When the same location on the body is exposed to sustained pressure and shear, a pressure ulcer can develop in that location. Pressure ulcers are painful wounds that can develop quickly but tend to heal slowly. Areas particularly prone to pressure ulcer development include the sacrum, coccyx, heels, elbows, and other bony prominences of the body.

In a hospital or care facility, caregivers adhere to specific protocols to prevent the occurrence of pressure ulcers. One important part in the prevention regimen is to offload the pressure exerted on the body.

Various types of support and offloading mattresses exist on the market, which aim to prevent the formation of pressure ulcers. Such mattresses may comprise e.g. a foam or air to redistribute pressure under the body.

Fluidized positioners may also be used to offload the pressure. A fluidized positioner is a large pressure redistribution cushion, which comprises a fluidized medium. In use, the fluidized positioner may be arranged directly underneath a body part of a patient in a hospital bed. Alternatively, it may be used in conjunction with a mattress to angle a patient and hold the patient in a specific position for the purpose of pressure offloading.

One challenge associated with pressure offloading mattresses and fluidized positioners is that when these products are used for pressure offloading, the mattress and the positioner may "bottom out".

Bottoming out means that when a heavy body part is immersed into the mattress or positioner for a prolonged period, the pressure offloading material contained therein has a tendency to flow towards the edges of the mattress or the fluidized positioner. As a result, the area of the mattress or positioner in contact with the heavy body part is left with a significantly reduced amount of pressure offloading material. This is not desired since the pressure offloading effect may then be impaired.

Another challenge with positioners is that they contain a plastic outer shell, which renders the positioner slippery. This may cause the positioner to slide against a bed surface and/or the mattress in use. In this regard, additional means may be required to hold the patient (and the positioner) in position.

Furthermore, both mattresses and positioners are bulky and large products and the pressure offloading effect is not always concentrated to the specific area in need of protection.

Accordingly, there is a need to provide an improved means for pressure offloading of a patient at risk of developing pressure ulcers. Such means should be convenient to use for the patient and for handling by the caregivers. Furthermore, such means should solve or at least alleviate the challenges mentioned hereinbefore.

### SUMMARY

In view of the above mentioned and other drawbacks of the prior art, it is an object of the present disclosure to provide improvements with respect to pressure offloading and to prevent pressure ulcers from occurring in the first place.

According to a first aspect of the present disclosure, there is provided a pressure offloading pad for incorporation into a garment; the pressure offloading pad comprising a skin-facing layer and a bladder, wherein the bladder comprises a pressure offloading material selected from air or a fluidized medium.

The present disclosure is based on the realization that the incorporation of a pressure offloading pad into a garment yields an improved and more concentrated pressure offloading effect. The pressure offloading pad is held in place in the garment such that the pressure offloading effect can be concentrated to a specific area or a specific bony prominence in need of protection.

For example, the garment may be an underwear garment or a sock such that the sacral region of a patient or the heel of a patient can be properly offloaded. The incorporation of the pressure offloading pad in the garment prevents the pad from sliding against the bed surface. Furthermore, it prevents the pad from bottoming out since the pad is constrained by the garment and is of a considerably smaller size.

The smaller size of the of the pressure offloading pad is also more convenient for the patient, and easier to handle by the caregivers.

The pressure offloading material is selected from air or a fluidized medium. These materials are beneficial to secure that the bony prominence or specific body part of a patient is properly offloaded.

In exemplary embodiments, the skin-facing layer comprises a spacer fabric material.

A spacer fabric material is a three-dimensional knitted fabric which comprises a top layer and a bottom layer being connected by an interconnecting layer of pile filaments. The pile filaments extend between the top layer and bottom layer and secure that a defined distance is established between the layers.

The provision of a spacer fabric material is beneficial from a microclimate point of view. The distance between the top layer and the bottom layer of the spacer fabric material provides for an improved ventilation of air between these layers. Sweat and moisture exerted from the body is transported away from the skin surface, and accumulation of body liquids close to the skin is thus prevented. Furthermore, heat generated may be wicked away from the skin.

The spacer fabric material also improves the pressure offloading effect. The spacer fabric material is resistant to compression and is configured to withstand pressures exerted on the pad during use. After a compressive force has been exerted to pad, the spacer fabric material is configured to return to its original shape immediately after removal of the force.

In exemplary embodiments, the skin-facing layer has a lateral (x) extension and a longitudinal (y) extension and wherein the skin-facing layer is stretchable in the longitudinal (y) and lateral (x) directions of extension.

This allows for the skin-facing layer to conform to the movement of a patient and to conform to any contoured shape adopted by the bladder of the pressure offloading pad.

Furthermore, friction against the skin (in cases where the skin-facing layer is in direct contact with the skin) is significantly reduced.

The stretchability of the skin-facing layer also prevents wrinkles and creases from forming, which may give rise to undesirable pressure points.

In exemplary embodiments, the pressure offloading material is a fluidized medium, wherein the fluidized medium has no shape memory.

Accordingly, the pressure offloading pad may easily adopt a specific shape with little force but retains its shape when the force is removed. This way, the pressure offloading pad may be sculpted and may provide three-dimensional contouring to a body part during use. The pressure offloading effect is thereby improved.

In exemplary embodiments, the fluidized medium comprises a viscous fluid and a plurality of microparticles.

The three-dimensional contouring and the moldability of the bladder are thus improved. The viscous fluid lubricates and coats the exterior surface of the microparticles and reduces the friction between the microparticles. The mixture of microparticles and the viscous fluid secure that a compact fluidized medium is provided, which does not flow in the absence of a force exerted on the pad.

The viscous fluid may be silicone oil or mineral oil.

In exemplary embodiments, the bladder is a first bladder and wherein the pressure offloading pad further comprises a second bladder; the first bladder having a first side arranged in contact with the skin-facing layer and a second side arranged in contact with the second bladder, wherein the first bladder comprises a fluidized medium and wherein the second bladder comprises air.

The first bladder comprising the fluidized medium is arranged proximal the skin surface and in contact with the skin-facing layer. This is beneficial to yield an improved micro-contouring against the skin of a patient. The second bladder comprises air and is arranged distal from the skin of the patient, and in proximity of e.g. the bed surface. In this regard, the air-filled second bladder provides an improved macro-contouring towards the bed surface (and also towards the underlying first bladder).

The configuration of having two bladders arranged on top of each other is beneficial as it yields a larger contact surface towards the body part immersed into the pressure offloading pad. The overall pressure offloading effect may thus be improved.

Furthermore, this configuration may also be beneficial from a cost perspective. A fluidized medium is typically an expensive material, and the combination with air may thus reduce the costs associated with manufacturing the pressure offloading pad since less material may be required in the second bladder.

In exemplary embodiments, the bladder comprises a first layer and a second layer; the first and second layers being connected along peripheral edges of the first and second layers, wherein the first and the second layer comprise a plurality of holes extending through the first and the second layer, and wherein the first and the second layers are connected along the perimeter of each hole.

For example, the first and the second layers may be connected by means of welded seams. Accordingly, the bladder may comprise a plurality of "weld-punched" holes.

The pressure offloading material; i.e. air or fluidized medium, is arranged in the areas of the pad surrounding the holes. When a heavy body part with a wide immersion area (e.g. the sacrum) is arranged in contact with the pressure offloading pad, the holes restrict, or at least significantly reduce and slow down, horizontal expansion of the pressure offloading material such that the material is kept in the area where it provides optimal pressure offloading. The holes may thus assist in preventing the pad from bottoming out in a specific area. In addition, the holes prevent vertical expansion of the pressure offloading material in a specific area of the pressure-offloading pad; i.e. at the peripheral edges of the bladder/pad.

Furthermore, the provision of holes in the bladder improves the breathability of the pressure offloading pad. In this regard, the overall microclimate of the product is improved.

According to another aspect, there is provided a garment comprising a pressure offloading pad as described hereinbefore.

The garment may be any garment intended to be worn over bony prominences of the body.

The pressure offloading pad may be integrated in the garment or it may be detachably attached to the garment.

For example, the pressure offloading pad may be sewn into the garment, and thus become an integral component of the garment.

Alternatively, the pressure offloading pad is detachably attached to the garment.

The detachable attachment is beneficial since the pressure offloading pad may easily be removed and re-inserted if the garment is to be washed or changed for some other reason.

For example, the garment may comprise a pocket in which the pressure offloading pad is arranged.

A pocket secures that the pressure offloading pad is held firmly in place during use. The size of the pocket typically matches the size of the pad. In this regard, the garment restricts the horizontal expansion of the pressure offloading material of the pad when e.g. a heavy body port is arranged in contact therewith. Furthermore, a pocket allows for the pressure offloading pad to be quickly and easily detached from and attached to the garment.

In exemplary embodiments, the garment is an underwear garment or an adult diaper and wherein the pressure offloading pad is arranged in an area of the underwear garment intended to contact the sacral region of a wearer.

The sacral region of a patient is particularly prone to developing pressure ulcers, especially when patients are being bed-ridden during long periods. The anatomy and physiology of the sacrum make the tissue very vulnerable to sustained pressure. As the patient moves or slides in bed, the soft tissue in the sacral region may be compressed, and shear forces may arise in the soft tissue. The incorporation of the pressure offloading pad in the underwear of a patient may thus significantly prevent pressure ulcers from occurring in this region, and secure that the sacral region, including the coccyx, is properly offloaded.

In exemplary embodiments, the garment is a sock and wherein the pressure offloading pad is arranged in an area of the sock intended to contact the heel of a wearer.

The heels are also regarded as a "high-risk" area for pressure ulcer formation. The heels comprise various bony prominences that need to be protected. A sock in accordance with the present disclosure secures that the heels are offloaded such that the risk of developing heel pressure ulcers is minimized.

In exemplary embodiments, the garment is an adult diaper, wherein the adult diaper is absorbent.

Patients suffering from (or at risk of developing) pressure ulcers are often bedridden during prolonged periods. These patients may also suffer from incontinence. Accordingly, the incorporation of a pressure ulcer prevention pad within the adult diaper combines the advantages of handling of urine and other body fluids, while simultaneously preventing pressure ulcers from forming.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1a schematically illustrates a patient wearing a garment comprising a pressure offloading pad according to an exemplary embodiment of the present disclosure.
Figure 1b is a partial, cross-sectional view of the pressure offloading pad incorporated into the garment of figure 1a.
Figure 2a illustrates a bladder of the pressure offloading pad comprising a plurality of holes according to an exemplary embodiment of the present disclosure.
Figure 2b is a partial, cross-sectional view of a pressure offloading pad incorporated into the garment of figure 1a and comprising the bladder of figure 2a.
Figure 3 illustrates a sock comprising a pressure offloading pad according to an exemplary embodiment of the present disclosure.

### DETAILED DESCRIPTION

The present invention will now be described more fully hereinafter with reference to the accompanying drawings, in which currently preferred embodiments of the present invention are shown. The present invention may, however, be embodied in many different forms and should not be construed as limited to the embodiments set forth herein; rather, these embodiments are provided for thoroughness and completeness, and fully convey the scope of the present invention to the skilled person. Like reference characters refer to like elements throughout.

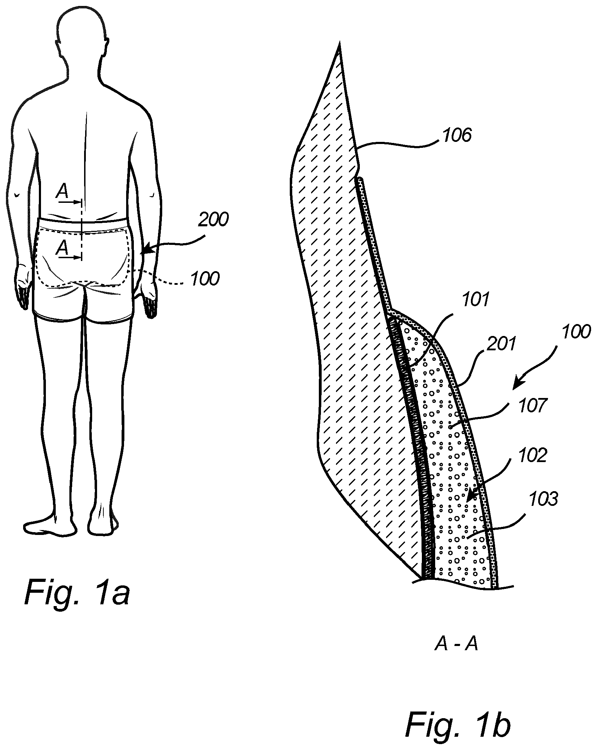

Figure 1a schematically illustrates a pressure offloading pad 100 incorporated into a garment 200. In figure 1a, the garment 200 is an underwear garment.

As best illustrated in figure 1b, the pressure offloading pad 100 comprises a skin-facing layer 101 and a bladder 102, wherein the bladder comprises a pressure offloading material 103 selected from air or a fluidized medium.

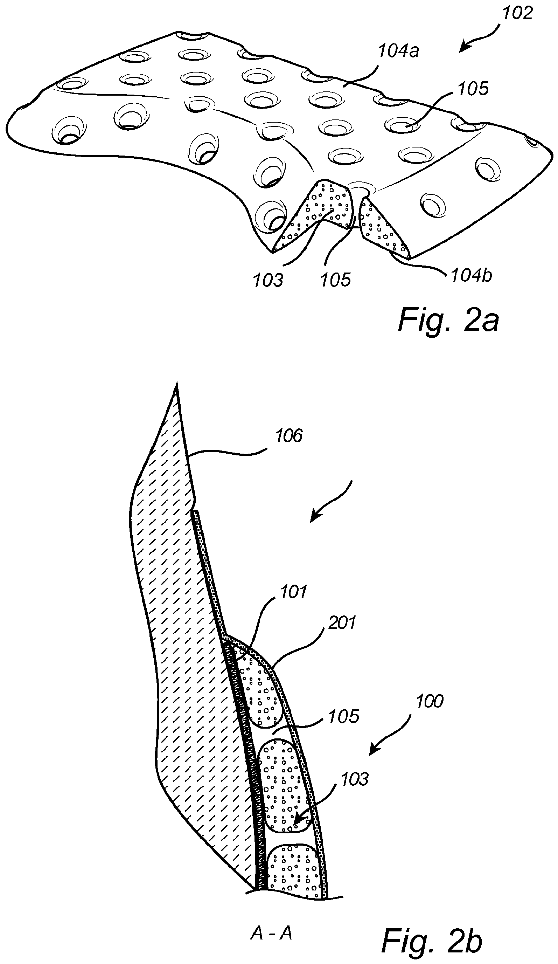

The "skin-facing layer" is arranged to face the skin of a patient. In figures 1b and 2b, the skin of the patient is denoted 106. In some embodiments, the skin facing layer may be skin-contact layer. The skin-facing layer is thus arranged in direct contact with the skin of a patient (as illustrated in figures 1b and 2b). Alternatively, the skin-facing layer is arranged in contact with a layer of the garment into which the pressure offloading pad is to be arranged. Consequently, it is equally conceivable that a layer of the garment is arranged in direct contact with the skin of the patient and that the skin-facing layer 101 is arranged in direct contact with such a garment layer.

The skin-facing layer may comprise any skin-friendly and breathable material. For example, the skin-facing layer may comprise a nonwoven material, an absorbent foam or a spacer fabric material.

The pressure offloading pad 100 comprises a bladder 102 housing the pressure offloading material.

As used herein, the term "bladder" means a sealed compartment or chamber comprising a pressure offloading material. The bladder may be formed by two layers being connected along their peripheral edges. The layers of the bladder typically comprise a moisture and vapor impermeable material. For example, the layers may comprise a plastic material such as a polymer-based material, e.g. polyurethane, polyethylene etc.

The bladder 102 may be formed by two polymer-based films, e.g. polyurethane films, welded together. The thickness of the layers forming the bladder may be in the range of from 0.05 to 0.5 mm, e.g. in the range of from 0.08 to 0.3 mm.

This range is desired to secure that the pressure offloading material is prevented from leaking out from the bladder and the pad during use. If the thickness of the layers forming the bladder is too high, this may restrict the ability of the pressure offloading pad to contour to the body part during use. If the thickness is too low, the risk of rupture of the bladder during use may be enhanced.

As mentioned hereinbefore, the skin-facing layer 101 preferably comprises a breathable material. When a patient is bedridden, excessive heat and moisture (e.g. sweat) may be generated. In this regard, the microclimate at the skin site typically affected and impaired. A breathable material improves the microclimate and the overall comfort for the patient.

As illustrated in e.g. figure 1b, the skin-facing layer 101 may comprise a spacer fabric material.

A "spacer fabric material" is a three-dimensional knitted fabric. The spacer fabric material comprises a top layer and a bottom layer being connected by an interconnecting layer of pile filaments. The pile filaments extend between the top layer and bottom layer and secure that a defined distance is established between the layers. The top layer of the spacer fabric layer is arranged distal from the skin of the patient in use. The bottom layer of the spacer fabric layer is arranged proximal to the skin of the patient in use. The top layer and the bottom layer of the spacer fabric are typically co-extensive; i.e. the top layer and the bottom layer have the same surface area.

The spacer fabric layer is not limited to a specific material. In exemplary embodiments, the spacer fabric layer comprises polyester. The pile filaments of the interconnecting layer may be polyester monofilament fibers.

The skin-facing layer 101 may be attached to the bladder 102 by adhesion or welding.

The pressure off-loading pad 100 may comprise additional layers that yield a comfort, absorbent, or pressure-offloading effect. For example, the pressure offloading pad 100 may comprise a nonwoven layer and/or a polyurethane foam. The additional layer(s) are typically arranged between the skin-facing layer 101 and the bladder 102.

It is also conceivable that the pressure offloading pad 100 comprises a cover layer. The bladder 101 has a first side facing the skin-facing layer 101 and an opposing second side. The cover, where present, may be arranged on the second side of the bladder.

The skin-facing layer 101 has a lateral (x) extension and a longitudinal (y) extension and wherein the skin-facing layer 101 may be stretchable in the longitudinal (y) and lateral (x) directions of extension.

Accordingly, the skin-facing layer 101 is configured to conform to the movement of the patient and to the bladder housing the pressure offloading material. Furthermore, the stretchable skin-facing layer reduces the friction against the skin and prevents wrinkles and creases from forming. Such wrinkles and creases may give rise to undesirable pressure points.

If the skin-facing layer is too stiff, this may restrict the ability of the bladder to contour to the body part or bony prominence of the patient in use.

The pressure offloading material 103 may be either air and/or a fluidized medium.

The filling degree of the pressure offloading material in the bladder may be in the range of from 50 to 95 %, e.g. from 60 to 80%.

A filling degree in the above-mentioned range is beneficial to allow the body part to immerse into the pressure-offloading material of the pad such that a large contact surface against that body part is provided.

As used herein, the term "filling degree" means the ratio of the pressure offloading material vs. the maximum internal volume of the bladder (at ambient pressure).

The filling degree may be in the lower range, e.g. from 50 to 70% if a heavy and larger body part is to be offloaded. The filling degree may be in the higher range, e.g. from 70 to 95% if a smaller body part, e.g. an elbow or a heel is to be offloaded.

These ranges are beneficial to secure proper pressure offloading.

The bladder may be filled with the pressure offloading material, and subsequently, the bladder may be sealed, e.g. by welding. Prior to sealing, a predetermined amount of gas, such as air, may be removed by vacuum. The removal of gas may be varied and may affect the flow characteristics of the fluidized medium. For example, gas may be evacuated to achieve a subatmospheric pressure in the range of 5 to 500 mbar below ambient pressure.

The maximum thickness of the bladder 102 may be in the range of from 10 to 200 mm, e.g from 20 to 100 mm.

The thickness will vary depending on the use of the pressure offloading pad, i.e. depending on what particular area to be offloaded and which type of garment that the pad is to be inserted into. For example, a pressure offloading pad intended to offload the back or sacral region of an adult patient may be thicker than a pad intended to offload the heel or the elbow.

The pressure offloading pad 100 may have a lateral extension; i.e. length, of from 10 cm to 50 cm, e.g. from 15 to 30 cm.

The pressure offloading pad 100 may have a longitudinal extension; i.e. width of from 8 cm to 30 cm, e.g. from 10 to 20 cm.

The width and the length of the pad will be different depending on the use of the pad; i.e. which body part is to be offloaded, and which type of garment the pad is to be used with.

As used herein, the term "fluidized medium" means a medium which flows under an applied shear force. The fluidized medium does not flow due to gravity. The fluidized medium is preferably shape retaining and may be pushed into a shape with very little force, but retain that shape when the force is removed.

The fluidized medium 102 comprises a viscous fluid, such as a viscous oil, e.g. silicone oil or a mineral oil. The fluidized medium may further comprise microparticles (denoted 107 in figure 1b). The microparticles may have an average diameter less than 500 µm, e.g. an average diameter of from 10 to 300 µm.

The viscous fluid lubricates and coats the exterior surface of the microparticles 107 and reduces the friction between the microparticles. A compact and firm medium, which does not flow in the absence of a shear force, is thus accomplished.

A viscous fluid means a fluid having a viscosity in the range of from 100 to 600 cSt, preferably from 200 to 400 cSt at a temperature of 20°C.

Preferably, the microparticles 107 are microspheres. The microspheres may be hollow phenolic, glass or plastic particles. Typically, the microspheres are hollow spherical particles with plastic walls.

The fluidized medium may further comprise an additional lightweight material, such as foam beads. The foam beads may e.g. comprise polyethylene or polystyrene.

In embodiments, where the pressure offloading material is a fluidized medium; the fluidized medium preferably has no shape memory.

Hence, after the moldable pad has adopted a specific shape upon the application of a force, that shape is retained when the force is removed. This is beneficial as the ability of the pressure offloading pad to contour to the bony prominence of the patient is significantly improved.

It is also conceivable that the pressure offloading pad 100 comprises two bladders arranged on top of each other.

For example, the bladder 102 may be a first bladder and wherein the pressure offloading pad further comprises a second bladder; the first bladder having a first side arranged in contact with the skin-facing layer 101 and a second side arranged in contact with the second bladder, wherein the first bladder comprises a fluidized medium and wherein the second bladder comprises air (not shown).

The first and the second bladder typically have the same surface area; i.e. the first and second bladders are coextensive in length and in width.

The first bladder is arranged proximal to the skin and in contact with the skin-facing layer. The second bladder is arranged distal from the skin of the patient and from the skin-facing layer.

The combination of a first bladder with a fluidized medium and a second, air-filled bladder, is beneficial to improve the overall pressure offloading effect, particularly when the pressure offloading pad is utilized for offloading a heavy body part, such as the sacrum or the back of a patient. A combined macro-contouring (by means of the second bladder) and micro-contouring (by means of the first bladder) effect can thus be achieved.

As best illustrated in figures 2a and 2b, the bladder 102 may comprise a first layer 104a and a second layer 104b; the first 104a and second 104b layers being connected along peripheral edges of the first and second layers 104a, 104b, wherein the first 104a and the second layer 104b comprise a plurality of holes 105 extending through the first 104a and the second 104b layer, and wherein the first 104a and the second 104b layers are connected along the perimeter of each hole 105.

The first 104a and the second 104b layers are typically connected by welded seams. The first 104a and the second 104b layers are thus welded along the perimeter of each hole and along the peripheral edges.

The pressure offloading material is restricted by the welded seams of the bladder and is also restricted by the holes which secure that the pressure offloading material remains in place and is maintained in the area underneath the body part. An improved cushioning effect is accomplished by the weld-punched holes. Furthermore, this configuration secures that the risk of bottoming out is significantly reduced when a heavy body part is arranged in contact with the pad.

The size of the holes and the density or distribution of the holes may be adjusted depending on the use of the pressure offloading pad; e.g. depending on what body part is to be offloaded etc.

According to another aspect, the present disclosure also relates to a garment comprising the pressure offloading pad 100 as described hereinbefore.

The garment may be any piece of clothing intended to be worn over a bony prominence.

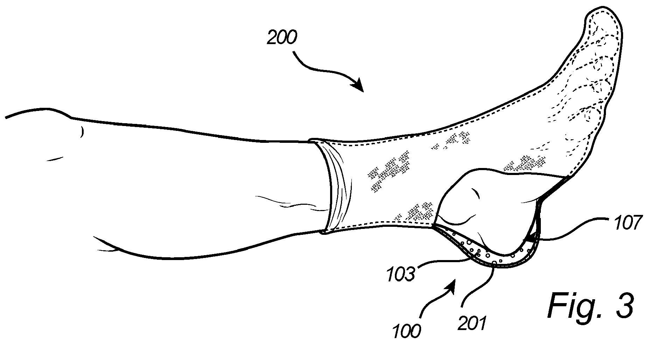

Preferably, the garment 200 is an underwear garment (see figure 1a), a sock (see figure 3) or an adult diaper (not shown).

The pressure offloading pad 100 may be integrated in the garment 200 or detachably attached to the garment 200.

Hence, the pressure offloading pad 100 may be integral with the garment 200. For example, the garment may be sewn into the garment.

It is, however, preferred that the pressure offloading pad 100 is detachably attached to the garment 200. This is beneficial since the pressure offloading pad 100 may easily be removed when the garment is to be washed or changed for some other reason, and subsequently be re-inserted in the garment.

The present disclosure is not limited to a particular mode of detachable attachment. For example, the pressure offloading pad may be detachably attached by a hook and loop attachment or any other mode of attachment.

Alternatively, the garment 200 may comprise a pocket in which the pressure offloading pad 100 is arranged.

A pocket is beneficial to secure that the pressure offloading pad is held firmly in place during use. The pocket may also constrain the pad and assist in preventing the pressure offloading pad from bottoming out.

The pocket may be defined by an inner garment layer and an outer garment layer. In figure 1b, the outer garment layer of the garment is denoted 201.

The garment 200 may be an underwear garment or an adult diaper and wherein the pressure offloading pad 100 is arranged in an area of the underwear garment intended to contact the sacral region of a wearer.

In figures 1a, and 2b, the garment is an underwear garment. However, in embodiments where the garment is an adult diaper, the adult diaper is typically a pant diaper. Hence, the adult diaper may generally have the same construction as the underwear garment illustrated in figures 1a and 2b.

The underwear garment may be a brief panty or a shorts panty.

The sacral region of a patient is particularly susceptible to pressure ulcer formation, and it is therefore important to provide proper pressure offloading in the sacral region of a patient.

As illustrated in figure 3, the garment may be a sock and wherein the pressure offloading pad 100 is arranged in an area of the sock intended to contact the heel 108 of a wearer.

The heel comprises various bony prominences particularly susceptible to pressure ulcer formation. Examples of such bony prominences include under the heel, the outside of the heel and the malleoli.

Preferably, the pressure offloading pad 100 is arranged to cover these bony prominences of the heel.

To date, heel boots may be utilized to offload the heels of a bed-ridden patient. However, conventional heel boots are typically large and bulky and suffer from poor breathability and a poor microclimate.

Alternatively, the nursing personnel may apply a wound dressing onto the heels of a patient to minimize the risk of pressure ulcer formation. However, a wound dressing is typically not designed to offload the pressure from the heel. Heel dressings are very challenging to apply and to hold in place since the heel consists of various convex parts that need to be covered, resulting in numerous directions and planes of extensions for the dressing. The heel is often referred to as a "hard to dress" area due to the difficulties in applying a dressing and securing that the wound dressing stays in place.

In contrast, the incorporation of a pressure offloading pad in a sock, according to the present disclosure, secures that the pad is held securely in place and that the pressure offloading effect is concentrated to the area in need of protection. Furthermore, the pressure offloading pad is more convenient to use for the patient and for handling by the caregivers (compared to e.g. heel boots).

In alternative embodiments, the garment may be an adult diaper, wherein the adult diaper is absorbent.

As used herein, the term "adult diaper" means a disposable adult incontinence diaper. Typically, the adult diaper is a pant-type diaper. The adult diaper typically comprises a topsheet, a backsheet and an absorbent member arranged between the topsheet and the backsheet. The absorbent member may be arranged in the crotch portion of the wearer.

An adult diaper comprising the pressure offloading pad may suitably be used with incontinent patients. For example, an adult diaper may be used during night time.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A pressure offloading pad (100) for incorporation into a garment (200); said pressure offloading pad (100) comprising a skin-facing layer (101) and a bladder (102), wherein said bladder comprises a pressure offloading material (103) selected from air or a fluidized medium.

2. The pressure offloading pad (100) according to claim 1, wherein said skin-facing layer (101) comprises a spacer fabric material.

3. The pressure offloading pad (100) according to claim 1 or claim 2, wherein said skin-facing layer (101) has a lateral (x) extension and a longitudinal (y) extension and wherein said skin-facing layer (101) is stretchable in the longitudinal (y) and lateral (x) directions of extension.

4. The pressure offloading pad (100) according to any one of the preceding claims, wherein said pressure offloading material (103) is a fluidized medium, wherein said fluidized medium has no shape memory.

5. The pressure offloading pad (100) according to any one of the preceding claims, wherein said pressure offloading material (103) is a fluidized medium comprising a viscous fluid and a plurality of microparticles (107).

6. The pressure offloading pad (100) according to claim 5, wherein said viscous fluid is silicone oil or mineral oil.

7. The pressure offloading pad (100) according to any one of the preceding claims, wherein said bladder (102) is a first bladder and wherein said pressure offloading pad further comprises a second bladder; said first bladder having a first side arranged in contact with said skin-facing layer (101) and a second side arranged in contact with said second bladder, wherein said first bladder comprises a fluidized medium and wherein said second bladder comprises air.

8. The pressure offloading pad (100) according to any one of the preceding claims, wherein said bladder (102) comprises a first layer (104a) and a second layer (104b); said first (104a) and second (104b) layers being connected along peripheral edges of said first and second layers (104a, 104b), wherein said first (104a) and said second layer (104b) comprises a plurality of holes (105) extending through said first (104a) and said second (104b) layer, and wherein said first (104a) and said second (104b) layers are connected along the perimeter of each hole (105).

9. A garment (200) comprising a pressure offloading pad (100) according to any one of claim 1-8.

10. The garment (200) according to any one of the preceding claims, wherein said garment (200) is an underwear garment, a sock, or an adult diaper

11. The garment (200) according to claim 9 or claim 10, wherein said pressure offloading pad (100) is integrated in said garment (200) or is detachably attached to said garment (200).

12. The garment (200) according to any one of the preceding claims, wherein said garment (200) comprises a pocket in which said pressure offloading pad (100) is arranged.

13. The garment (200) according to any one of claims 9-12, wherein said garment (200) is an underwear garment or an adult diaper and wherein said pressure offloading pad (100) is arranged in an area of said underwear garment intended to contact the sacral region of a wearer.

14. The garment (200) according to any one of claims 9-12, wherein said garment is a sock and wherein said pressure offloading pad (100) is arranged in an area of said sock intended to contact the heel of a wearer.

15. The garment (200) according to any one of the preceding claims, wherein said garment (200) is an adult diaper, and wherein said adult diaper is absorbent.
